# EUROPEAN PATENT APPLICATION

(11) **EP 2 022 435 A1**
(43) Date of publication of application: **11.02.2009**
(21) Application number: 08010994.5
(22) Date of filing: 17.06.2008
(51) Int. Cl.: A61B 19/02, A61G 13/00, A61G 12/00

(54) **Medical servicing system**

(30) Priority: 26.07.2007 US 828762
(71) Applicant: Berchtold Holding GmbH, 78532 Tuttlingen (DE)
(72) Inventor: Nies, John H., III, Mount Pleasant, SC 29466 (US); Hand, Barry, Mount Pleasant, SC 29464 (US); Conly, Chris, Hanahan, SC 29410 (US)
(74) Representative: Manitz, Finsterwald & Partner GbR

(57) **Abstract**

A catheter assembly (20) and method is disclosed which comprises a catheter (22) having a coating on at least a part of its length intended to produce a low-friction surface on the catheter when treated with an activating substance in the form of a vapor. The catheter assembly (20) also includes a catheter package (24) forming an interior space divided by a gas permeable, liquid impermeable barrier (28) into a first cavity (26a) and a second cavity (26b). The first cavity (26a) of the catheter package accommodates the catheter therein and the second cavity (26b) accommodates at least a quantity of liquid (30) in its liquid phase therein. The liquid (30) in the second cavity can change phase into a vapor capable of passing from the second cavity, through the barrier (28) and into the first cavity (26a), where the vapor can activate the coating. As a result, the vapor produced when the liquid changes phase causes the coating on the catherer to be activated to thereby produce the low-friction surface on the catherer (22) so it is ready-to-use when it reaches the end user.

## Description

### FIELD

The present disclosure relates to medical servicing systems and, more particularly, to an improved ceiling or wall mounted medical servicing system having an integrated equipment support assembly.

### BACKGROUND

The statements in this section merely provide background information related to the present disclosure and may not constitute prior art.

Medical practitioners and hospitals strive to reduce the time required to safely perform medical procedures, while reducing the amount of space required to locate necessary medical equipment at or near an operating theater. The time reduction is beneficial to the patient by reducing the possibility of surgical complications, and beneficial to the hospital by allowing more procedures to be preformed in a given day. The space reduction enables additional medical staff to have improved access to a patient during an operation. Accordingly, there is a consistent need to increase the efficiency of the operating theater before, during and after a medical procedure.

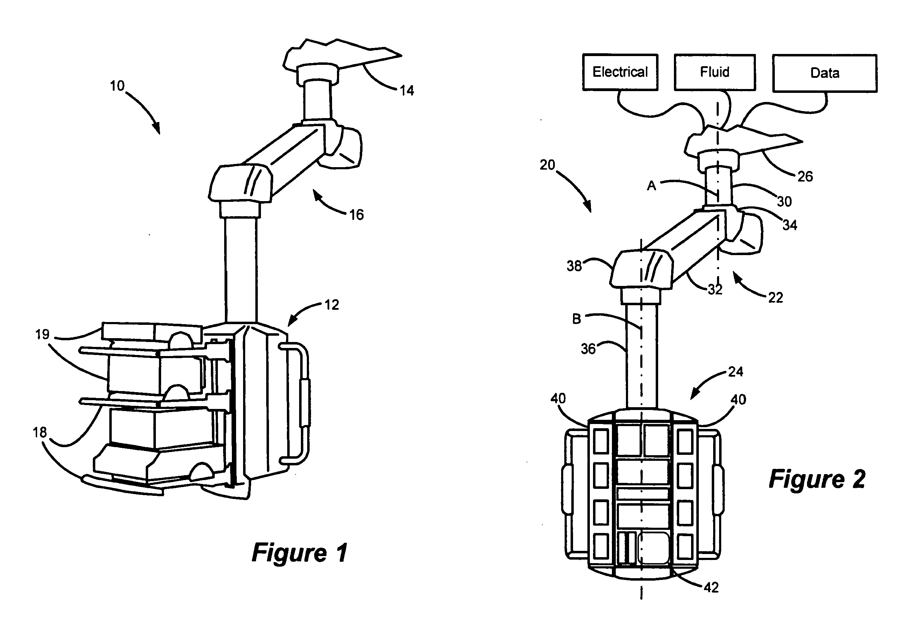

Referring now to Figure 1, a known medical servicing system 10 is illustrated. The medical servicing system 10 includes a service module 12 that is supported on a ceiling 14 by an overhead suspension system 16. The medical servicing system 10 is disclosed in U.S. Pat. Publication No. US 2003/0076015 A1, the disclosure of which is expressly incorporated herein by reference. The service module 12 includes a plurality of adjustable shelves 18, on which medical equipment 19 is supported. Although the medical servicing system 10 of Figure 1 improves the operating theater efficiency in comparison to previously known systems, the service module 12 is larger and is less efficient than desired.

There remains a need for a medical servicing system that is easy to operate, is able to efficiently position, organize, and support medical instruments and services, is adaptable to support today's and tomorrow's technology, and allows efficient movement of the supported instruments and services before, during and after a medical procedure.

### SUMMARY

Accordingly, the present disclosure provides a servicing system. The servicing system includes a suspension system connected to a support structure and a service module coupled to the suspension system. The service module includes a rack having a plurality of frame members coupled to one another to define module support openings. The module support openings include respective standard widths and respective standard heights. At least one equipment module is secured to the rack within a corresponding one of the module support openings.

In other features, the servicing system further includes a power source interface, to which the at least one equipment module is coupled to provide power thereto. The power source interface is coupled to a remote power source through the suspension system. The servicing system further includes a plurality of equipment modules that are commonly coupled to the power source interface.

In other features, the servicing system further includes a fastening member for securing the at least one equipment module to the rack. The fastening member includes a quick release fastening member.

In other features, the suspension system provides a path for at least one electric line, at least one fluid line and at least one data line. The service module includes at least one connector for each of the at least one electric line, at least one fluid line and at least one data line. The service module includes a plurality of panels and at least one of the panels includes a plurality of the connectors.

In still another feature, the service module includes at least one handle.

In yet another feature, the standard height conforms to an increment of a U rack standard and the standard width conforms to an increment of an HP rack standard.

The invention is particularly applicable for use in operating rooms during surgical procedures or the like and will be described with reference thereto. However, it is to be understood that the present invention is useful in a variety of situations, environments, and applications wherever electrical, pneumatic, or other equipment is needed, including non-medical uses and environments such as industrial and commercial applications.

Further areas of applicability will become apparent from the description provided herein. It should be understood that the description and specific examples are intended for purposes of illustration only and are not intended to limit the scope of the present disclosure.

### DRAWINGS

The drawings described herein are for illustration purposes only and are not intended to limit the scope of the present disclosure in any way.

Figure 1 is a perspective view of a traditional medical service system;

Figure 2 is a perspective view of a medical service system in accordance with the present disclosure;

Figure 3 is a front view of a service module of the medical service system of Figure 2;

Figure 4 is a side view of the service module of the medical service system of Figure 2; and

Figure 5 is a schematic illustration of a standardized rack for the medical service system.

### DETAILED DESCRIPTION

The following description is merely exemplary in nature and is not intended to limit the present disclosure, application, or uses. It should be understood that throughout the drawings, corresponding reference numerals indicate like or corresponding parts and features.

Referring now to Figure 2, a perspective view of a medical servicing system 20 in accordance with the present disclosure is shown. The present disclosure is particularly applicable for use in operating rooms during surgical procedures or the like, and will be described with reference thereto. However, it is to be understood that the present disclosure is useful in a variety of situations, environments and applications wherever electrical, pneumatic, or other equipment is needed, including non-medical uses and environments such as industrial and commercial applications. Further, the term servicing system, as defined herein, includes any system that facilitates or assists in providing desired services, such services including, but not limited to, positioning, organizing, supporting, or moving equipment and delivering electricity, fluid, or data to a desired location.

The medical servicing system 20 includes an overhead suspension system 22 having a service module 24 attached thereto. The medical servicing system 20 positions, organizes, supports and moves commonly used medical equipment and delivers electricity, fluid and/or data to desired locations in an operating theater. The overhead suspension system 22 is fixedly secured to structural reinforcement members in a ceiling 26, or to other suitable supports, such as, for example, a wall of an operating room or the like. The service module 24 is attached at a distal end of the overhead suspension system 22. The overhead suspension system 22 includes a mount (not shown) for connecting the overhead suspension system 22 to the ceiling 26. The mount includes any suitable fastening arrangement, including, for example bolts for securing overhead suspension system 22 to the ceiling 26.

The overhead suspension system 22 further includes a vertical connector 30 that is rotatably coupled to the mount and is rotatable about a vertical axis A. An arm 32 is coupled to the vertical connector 30 by a first joint 34. A vertical coupling 36 member is attached to a distal end of the arm 32 by a second joint 38. The service module 24 is attached to a distal end of the vertical coupling member 36 and is rotatable about a vertical axis B. Coupling of the various components of the overhead suspension system 22 is provided in any conventional manner, as long as the required degrees of freedom are provided. Further, each of the components of the overhead suspension system 22 can be held in position in a conventional manner using pneumatic brakes or the like, for example.

An internal conduit (not shown) is defined through the overhead suspension system 22 for accommodating one or more electric lines, fluid lines and/or data lines, which run from the ceiling 22 to the service module 24. The electric, fluid and/or data lines deliver electricity, fluid and/or data, respectively to and from patients, medical equipment and/or information systems supported in the service module 24. For example, low and high voltage can be carried along electric lines, a vacuum, gases such as NO₂, O₂, CO₂, HeO₂ and/or N₂, or liquid, may be supplied along fluid lines. Video, telephone or other data can be carried along the data lines. It is appreciated that other types of lines useful in the given environment can run through the internal conduit from the ceiling 26 to the service module 24. The electric, fluid and data lines may alternatively be run along the outside surface of overhead suspension system 22 to the service module 24 instead of through an internal conduit.

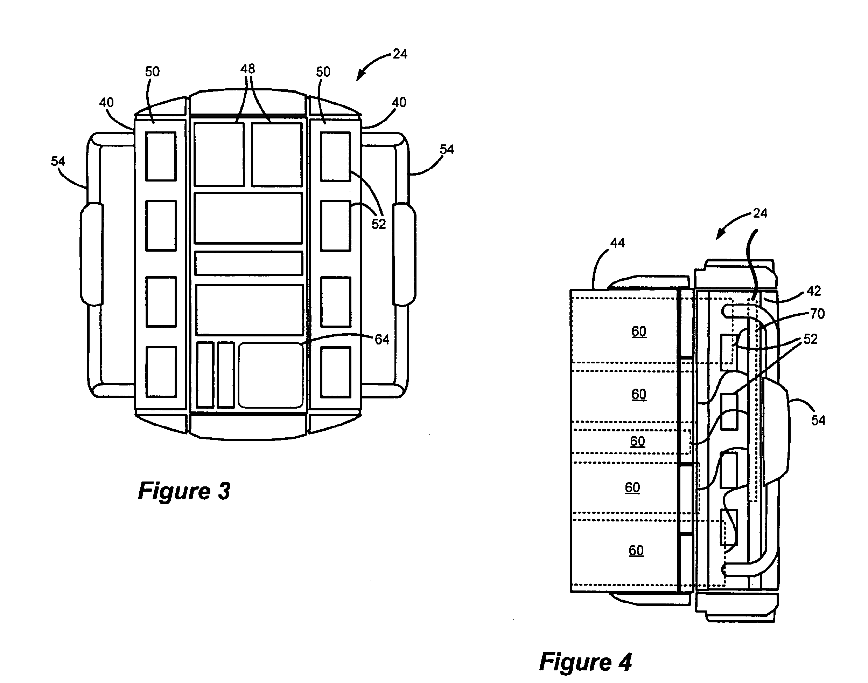

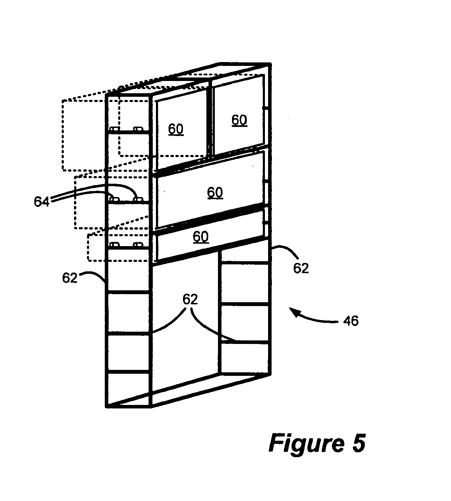

Referring now to Figures 2 - 5, an exemplary embodiment of the service module 24 of the present disclosure will be described. The service module 24 is configured about the vertical axis B and includes side panels 40, a front face 42 and a rear cover 44. A standardized rack system 46, discussed in further detail below, is enclosed within the service module 24. The front face 42 includes a first plurality of openings 48 in a central portion and two opposing angled panels 50 having a second plurality of openings 52. The service module 24 may include handles 54 attached to one or both of side panels 40. The handles 54 enable a medical practitioner or assistant to move service module 54 along the degrees of freedom provided by the overhead suspension system 22. For example, the service module 24 may be moved into and out of the surgical field, toward or away from the operating table or into any other position as desired.

The service module 24 of the present disclosure could alternatively be formed of any number of different shapes, such as generally rectangular or elliptical. Further, the service module 24 may be formed of any appropriate material that is capable of supporting any required loads. An exemplary material includes aluminum.

Each side panel 40 and angled panel 50 may include one or more electrical connectors, fluid connectors and/or data connectors that are fixed within the openings 48, 52. The electrical, fluid and/or data connectors are coupled to their respective sources via the lines, which extend from the ceiling 26. Any combination of electrical connectors, fluid connectors and/or data connectors may be provided on a respective side or angled panel 40, 50 of the service module 24. For example, the connectors can be arranged so that only one type of connector is located on any one panel. Further, the panels may include further connectors or devices, for example, a power switch, a phone connector, a CO₂ bottle holder, an arm member for a flat panel display, or an air vent or filtering device. The panels may be interchangable about the service module 24 and/or may include a hinge for allowing easy access to a rear portion of the connectors. The rear cover 44 is removable to provide access to the interior of the service module 24.

The front face 42 is fixedly attached to the standardized rack 46, which is disposed within the service module 24. The standardized rack 46 provides an interface for modularized servicing equipment. More specifically, the rack 46 can conform to a known standard such as a DIN (German Institute for Standardization) standard, for example. The rack 46 is housed within the servicing module 24 and is configured to support a plurality of medical equipment modules 60. These modules 60 include, but are not limited to, a light source module, an insufflator module, a camera module, an electrosurgical unit (ESU), an arthro pump and/or an argon beam coagulator, as a few examples.

The rack 46 includes a plurality of frame members 62 that are joined together to define a framework, which supports the various modules 60. The framework preferably conforms to fixed height and width increments provided at a given standard. For example, heights are provided in increments of the rack standard "U", where U is equal to 1.75 inches. This standard includes 1U (1.75 inches), 3U (5.25 inches), 6U (10.5 inches) and 9U (15.75 inches), for example. Widths are provided in increments of the rack standard HP, where HP is equal to 0.200 inches. In the case where a 3U height standard is used, the modules 60 can be 3U in height and in multiples of HP wide. In spaces where no module is present, a cover plate 64 is provided. The total dimensions of the rack 46 will depend on the size and number of modules 60 integrated therein.

Fasteners 64 are provided to secure the modules 60 to the frame members 62. The fasteners 64 can include traditional fasteners such as screws and/or bolts. It is also anticipated that the fasteners 64 can include quick release type fasteners such as clips and pins, for example. Such quick release fasteners enable the modules 60 to be rapidly and easily removed from and inserted into the service module 24.

A single power supply interface 70 is provided within the service module 24, to which each of the modules 60 connects. In an alternative embodiment, the common power supply can be located above the ceiling or in another location outside of the service module 24. The power supply interface 70 is coupled to a power source, for example, an AC or DC power source, through the overhead suspension system 22. In this manner, none of the modules 60, which are supported in the rack 46 within the service module 24, are provided with their own power supplies. As a result, the individual modules 60 can be smaller, lighter and less expensive to manufacture. In a further embodiment, a shelf (not shown) can be provided either above or below the rack 46. In this manner, a non-modularized medical equipment component, an ESU, for example, can be supported on the service module 24. It is anticipated that other, common interfaces can be provided including a data interface and/or a fluid interface, to which the individual modules 60 can be coupled and provided with data and/or fluid, respectively.

Through the servicing module 24 of the present disclosure, the efficiency in the use of the operating theater is improved by properly positioning, organizing, supporting, connecting and moving commonly used medical equipment and services required for a particular medical procedure. For example, the operating theater can be properly configured to easily and centrally locate devices such as display monitors (that require connection with data lines feeding video) with other pneumatic, hydraulic and/or electrically driven equipment. Further, the service module 24 of the present disclosure provides a smaller footprint within the operating area, thereby providing additional real estate within the vicinity of a patient for medical professionals. Because the integrated modules 60 do not require individual power supplies, the overhead suspension system 22 and the ceiling structure for anchoring the overhead suspension system 22 can be lighter and less expensive. The integrated modularity of the service module 24 enables the modules 60 to be easily replaced and reconfigured depending upon which modules 60 are required for a given surgery, and/or in the case where a module 60 must be removed for maintenance purposes.

Those skilled in the art can now appreciate from the foregoing description that the broad teachings of the present invention can be implemented in a variety of forms. Therefore, while this invention has been described in connection with particular examples thereof, the true scope of the invention should not be so limited since other modifications will become apparent to the skilled practitioner upon a study of the drawings, the specification and the following claims.

## Claims

1. A servicing system, comprising:
a suspension system connected to a support structure; and
a service module coupled to the suspension system, said service module comprising:
a rack having a plurality of frame members coupled to one another to define module support openings, wherein said module support openings include respective standard widths and respective standard heights; and
at least one equipment module that is secured to said rack within a corresponding one of said module support openings.

2. The servicing system of claim 1 further comprising a power source interface, to which said at least one equipment module is coupled to provide power thereto, wherein said power source interface is coupled to a remote power source through said suspension system.

3. The servicing system of claim 1 or 2 further comprising a plurality of equipment modules that are commonly coupled to said power source interface.

4. The servicing system of any of the preceding claims further comprising a fastening member for securing said at least one equipment module to said rack.

5. The servicing system of claim 4 wherein said fastening member includes a quick release fastening member.

6. The servicing system of any of the preceding claims wherein said suspension system provides a path for at least one electric line, at least one fluid line and at least one data line, and wherein said service module comprises at least one connector for each said at least one electric line, at least one fluid line and at least one data line.

7. The servicing system of claim 6 wherein said service module comprises a plurality of panels and at least one of the panels comprises a plurality of said connectors.

8. The servicing system of any of the preceding claims wherein said service module comprises at least one handle.

9. The servicing system of any of the preceding claims wherein said standard height conforms to an increment of a U rack standard and said standard width conforms to an increment of an HP rack standard.

10. A medical servicing system, comprising:
a suspension system connected to a support structure; and
a service module coupled to the suspension system, said service module comprising:
a rack having a plurality of frame members coupled to one another to define module support openings, wherein said module support openings include respective standard widths and respective standard heights, said respective standard heights conforming to an increment of a U rack standard and said respective standard widths conforming to an increment of an HP rack standard;
at least one equipment module that is secured to said rack within a corresponding one of said module support openings; and
a power source interface, to which said at least one equipment module is coupled to provide power thereto, wherein said power source interface is coupled to a remote power source through said suspension system.

11. The medical servicing system of claim 10 further comprising a plurality of equipment modules that are commonly coupled to said power source interface and / or further comprising at least one handle.

12. The medical servicing system of claim 10 or 11 further comprising a fastening member for securing said at least one equipment module to said rack.

13. The medical servicing system of claim 12 wherein said fastening member includes a quick release fastening member.

14. The medical servicing system of claim 10, 11, 12 or 13 wherein said suspension system provides a path for at least one electric line, at least one fluid line and at least one data line, and wherein said service module comprises at least one connector for each said at least one electric line, at least one fluid line and at least one data line.

15. The medical servicing system of claim 14 wherein said service module comprises a plurality of panels and at least one of the panels comprises a plurality of said connectors.
